# EUROPEAN PATENT SPECIFICATION

(11) **EP 2 068 956 B1**
(45) Date of publication and mention of the grant of the patent: **01.03.2017**
(21) Application number: 07796289.2
(22) Date of filing: 19.06.2007
(51) Int. Cl.: A61L 27/50, A61L 27/14, A61L 27/54, A61L 31/04, A61L 31/14, A61L 31/16, A61L 29/14, A61M 27/00

(54) **MEDICAL DEVICES FOR RELEASE OF LOW SOLUBILITY THERAPEUTIC AGENTS**
MEDIZINPRODUKTE ZUR FREISETZUNG VON THERAPEUTISCHEN MITTELN GERINGER LÖSLICHKEIT
DISPOSITIFS MÉDICAUX POUR LIBÉRATION D'AGENTS THÉRAPEUTIQUES DE FAIBLE SOLUBILITÉ

(30) Priority: 26.06.2006 US 474850
(43) Date of publication of application: 17.06.2009
(73) Proprietor: Boston Scientific Limited, Hamilton HM11 (BM)
(72) Inventor: BUCAY-COUTO, Weenna, Burlington, MA 01803 (US); LI, Jianmin, Lexington, MA 02421 (US); SHEU, Min-shyan, Chelmsford, MA 01824 (US)
(74) Representative: Pfenning, Meinig & Partner mbB
(86) International application number: PCT/US2007/014361
(87) International publication number: WO 2008/002434

(56) References cited:
- WO-A-2004/002447
- WO-A1-97/33552
- WO-A2-00/41687
- WO-A2-2005/087135
- US-A1- 2004 265 355

## Description

### FIELD OF THE INVENTION

The present invention relates generally to medical devices, and more particularly to implantable or insertable medical devices which release low-solubility therapeutic agents.

### BACKGROUND OF THE INVENTION

Controlled release of therapeutic agents by means of polymeric materials has existed in various forms for many years. For example, numerous polymer-based medical devices have been developed for the delivery of therapeutic agents to the body. Examples include drug eluting coronary stents, which are commercially available from Boston Scientific Corp. (TAXUS), Johnson & Johnson (CYPHER), and others.

The delivery of many drugs, however, has been complicated by their solubility within polymeric materials and/or body fluids. For example, many therapeutic agents are hydrophobic compounds which have low solubility in body fluids such as blood and urine, among others. Consequently, rather than being released, these therapeutic agents may be preferably retained within the polymeric material of the device. This is sometimes a limiting factor for delivering a therapeutically effective amount of therapeutic agent from the device to the target site.

US 2004 /0265355 A1 discloses medical devices for controlled drug delivery in the form of a matrix reinforced with fibers. WO 2004/002447 A2 relates to dosage forms in oral drug delivery systems providing increased solubility of drug compositions.

WO 2005/087135 A2 discloses a urological device coated with EVA and releasing antimicrobial agent having low solubility.

### SUMMARY OF THE INVENTION

According to an aspect of the present invention, implantable or insertable medical devices are provided, which contain one or more polymeric carrier regions. These polymeric carrier regions, in turn, deliver low solubility therapeutic agents with the assistance of solubilizing agents.

Advantages of the present invention are that polymeric carrier regions may be formed which release enhanced levels of low solubility therapeutic agents.

These and other aspects, embodiments and advantages of the present invention will become immediately apparent to those of ordinary skill in the art upon review of the Detailed Description and Claims to follow.

### BRIEF DESCRIPTION OF THE DRAWING

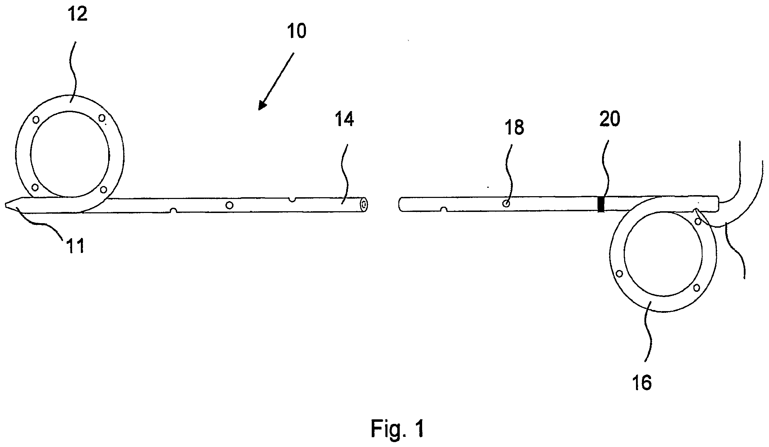

Fig. 1 is a side view of a ureteral stent, in accordance with an embodiment of the invention.

### DETAILED DESCRIPTION OF THE INVENTION

A more complete understanding of the present invention is available by reference to the following detailed description of numerous aspects and embodiments of the invention. The detailed description of the invention which follows is intended to illustrate but not limit the invention.

In one aspect, the present invention provides implantable or insertable medical devices, which contain one or more polymeric carrier regions. These polymeric carrier regions, in turn, contain (a) at least one polymer, (b) at least one therapeutic agent having a solubility in aqueous solution (e.g., distilled water, physiological saline, phosphate buffered saline, biological fluids including body fluids such as urine, blood, etc.) at 37°C of less than or equal to 1 mg/ml (referred to herein as "low solubility therapeutic agents"), for example, ranging from 1 mg/ml to 0.1 mg/ml to 0.01 mg/ml to 0.001 mg/ml or less, and (c) at least one solubilizing agent, as defined in the claims.

By "polymeric region" is meant a region (e.g., a device coating layer, a device component, an entire device, etc.) that contains one or more types of polymers. By "carrier region" is meant a region that contains one or more therapeutic agents. By "polymeric carrier region" is meant a region that contains one or more polymers and one or more therapeutic agents.

Medical devices benefiting from the present invention include a variety of medical devices, which are implanted or inserted into a subject, either for procedural uses or as implants. Examples include catheters (e.g., urinary or vascular catheters such as balloon catheters), guide wires, balloons, filters (e.g., vena cava filters), stents (including coronary artery stents, peripheral vascular stents such as cerebral stents, urethral stents, ureteral stents, biliary stents, tracheal stents, gastrointestinal stents and esophageal stents), stent grafts, vascular grafts, vascular access ports, embolization devices including cerebral aneurysm filler coils (including Guglilmi detachable coils and metal coils), myocardial plugs, pacemaker leads, left ventricular assist hearts and pumps, total artificial hearts, heart valves, vascular valves, tissue bulking devices, tissue engineering scaffolds for cartilage, bone, skin and other in vivo tissue regeneration, slings, sutures, suture anchors, anastomosis clips and rings, tissue staples and ligating clips at surgical sites, cannulae, metal wire ligatures, orthopedic prosthesis such as bone grafts, bone plates, joint prostheses, as well as various other medical devices that are adapted for implantation or insertion into the body.

The medical devices of the present invention include implantable and insertable medical devices that are used for systemic treatment, as well as those that are used for the localized treatment of any tissue or organ. Non-limiting examples are tumors, organs including the heart, coronary and peripheral vascular system (referred to overall as "the vasculature"), the urogenital system, including kidneys, bladder, urethra, ureters, prostate, vagina, uterus and ovaries, eyes, lungs, trachea, esophagus, intestines, stomach, brain, liver and pancreas, skeletal muscle, smooth muscle, breast, dermal tissue, cartilage, tooth and bone.

As used herein, "treatment" refers to the prevention of a disease or condition, the reduction or elimination of symptoms associated with a disease or condition, or the substantial or complete elimination of a disease or condition. Preferred subjects (also referred to as "patients") are vertebrate subjects, more preferably mammalian subjects and more preferably human subjects. Specific examples of medical devices for use in conjunction with the present invention include stents, such ureteral stents, which deliver one or more therapeutic agents into the urinary tract (e.g., for the treatment of pain and/or infection) or coronary stents and cerebral stents, which deliver one or more therapeutic agents into the vasculature (e.g., for the treatment of restenosis), among may other devices.

In some embodiments, the polymeric carrier regions of the present invention correspond to an entire medical device (e.g., in the form of a polymeric stent body that is loaded with therapeutic agent). In other embodiments, the polymeric carrier regions correspond to one or more portions of a medical device. For instance, the polymeric carrier regions can be in the form of medical device components, in the form of one or more fibers which are incorporated into a medical device, in the form of one or more polymeric layers formed over all or only a portion of an underlying medical device substrate, and so forth. Layers can be provided over an underlying substrate at a variety of locations and in a variety of shapes (e.g., in the form of a series of rectangles, stripes, or any other continuous or non-continuous pattern). As used herein a "layer" of a given material is a region of that material whose thickness is small compared to both its length and width. As used herein a layer need not be planar, for example, taking on the contours of an underlying substrate. Layers can be discontinuous (e.g., patterned). Terms such as "film," "layer" and "coating" may be used interchangeably herein.

Materials for use as underlying medical device substrates include ceramic, metallic and polymeric substrates. The substrate material can also be a carbon- or silicon-based material.

As noted above, polymeric carrier regions in accordance with the present invention contain at least one polymer, at least one low solubility therapeutic agent, and at least one solubilizing agent.

"Therapeutic agents," "drugs," "pharmaceutically active agents," "pharmaceutically active materials," and other related terms may be used interchangeably herein.

Exemplary therapeutic agents for use in conjunction with the present invention include the following (some of which are low solubility therapeutic agents, others of which are not): (a) anti-thrombotic agents such as heparin, heparin derivatives, urokinase, and PPack (dextrophenylalanine proline arginine chloromethylketone); (b) anti-inflammatory agents such as dexamethasone, prednisolone, corticosterone, budesonide, estrogen, sulfasalazine and mesalamine; (c) antineoplastic/ antiproliferative/anti-miotic agents such as paclitaxel, 5-fluorouracil, cisplatin, vinblastine, vincristine, epothilones, endostatin, angiostatin, angiopeptin, monoclonal antibodies capable of blocking smooth muscle cell proliferation, and thymidine kinase inhibitors; (d) anesthetic agents such as lidocaine, bupivacaine and ropivacaine; (e) anticoagulants such as D-Phe-Pro-Arg chloromethyl ketone, an RGD peptide-containing compound, heparin, hirudin, antithrombin compounds, platelet receptor antagonists, antithrombin antibodies, anti-platelet receptor antibodies, aspirin, prostaglandin inhibitors, platelet inhibitors and tick antiplatelet peptides; (f) vascular cell growth promoters such as growth factors, transcriptional activators, and translational promotors; (g) vascular cell growth inhibitors such as growth factor inhibitors, growth factor receptor antagonists, transcriptional repressors, translational repressors, replication inhibitors, inhibitory antibodies, antibodies directed against growth factors, bifunctional molecules consisting of a growth factor and a cytotoxin, bifunctional molecules consisting of an antibody and a cytotoxin; (h) protein kinase and tyrosine kinase inhibitors (e.g., tyrphostins, genistein, quinoxalines); (i) prostacyclin analogs; (j) cholesterol-lowering agents; (k) angiopoietins; (1) antimicrobial agents such as triclosan, cephalosporins, aminoglycosides and nitrofurantoin; (m) cytotoxic agents, cytostatic agents and cell proliferation affectors; (n) vasodilating agents; (o) agents that interfere with endogenous vasoactive mechanisms; (p) inhibitors of leukocyte recruitment, such as monoclonal antibodies; (q) cytokines; (r) hormones; (s) inhibitors of HSP 90 protein (i.e., Heat Shock Protein, which is a molecular chaperone or housekeeping protein and is needed for the stability and function of other client proteins/signal transduction proteins responsible for growth and survival of cells) including geldanamycin, (t) smooth muscle relaxants such as alpha receptor antagonists (e.g., doxazosin, tamsulosin, terazosin, prazosin and alfuzosin), calcium channel blockers (e.g., verapimil, diltiazem, nifedipine, nicardipine, nimodipine and bepridil), beta receptor agonists (e.g., dobutamine and salmeterol), beta receptor antagonists (e.g., atenolol, metaprolol and butoxamine), angiotensin-II receptor antagonists (e.g., losartan, valsartan, irbesartan, candesartan and telmisartan), and antispasmodic/anticholinergic drugs (e.g., oxybutynin chloride, flavoxate, tolterodine, hyoscyamine sulfate, diclomine), (u) bARKct inhibitors, (v) phospholamban inhibitors, (w) Serca 2 gene/protein, (x) immune response modifiers including aminoquizolines, for instance, imidazoquinolines such as resiquimod and imiquimod, and (y) human apolioproteins (e.g., AI, All, AIII, AIV, AV, etc.).

Numerous therapeutic agents, not necessarily exclusive of those listed above, have been identified as candidates for vascular treatment regimens, for example, as agents targeting restenosis. Such agents are useful for the practice of the present invention and include one or more of the following: (a) Ca-channel blockers including benzothiazapines such as diltiazem and clentiazem, dihydropyridines such as nifedipine, amlodipine and nicardapine, and phenylalkylamines such as verapamil, (b) serotonin pathway modulators including: 5-HT antagonists such as ketanserin and naftidrofuryl, as well as 5-HT uptake inhibitors such as fluoxetine, (c) cyclic nucleotide pathway agents including phosphodiesterase inhibitors such as cilostazole and dipyridamole, adenylate/Guanylate cyclase stimulants such as forskolin, as well as adenosine analogs, (d) catecholamine modulators including α-antagonists such as prazosin and bunazosine, β-antagonists such as propranolol and α/β-antagonists such as labetalol and carvedilol, (e) endothelin receptor antagonists, (f) nitric oxide donors/releasing molecules including organic nitrates/nitrites such as nitroglycerin, isosorbide dinitrate and amyl nitrite, inorganic nitroso compounds such as sodium nitroprusside, sydnonimines such as molsidomine and linsidomine, nonoates such as diazenium diolates and NO adducts of alkanediamines, S-nitroso compounds including low molecular weight compounds (e.g., S-nitroso derivatives of captopril, glutathione and N-acetyl penicillamine) and high molecular weight compounds (e.g., S-nitroso derivatives of proteins, peptides, oligosaccharides, polysaccharides, synthetic polymers/oligomers and natural polymers/oligomers), as well as C-nitroso-compounds, O-nitroso-compounds, N-nitroso-compounds and L-arginine, (g) ACE inhibitors such as cilazapril, fosinopril and enalapril, (h) ATII-receptor antagonists such as saralasin and losartin, (i) platelet adhesion inhibitors such as albumin and polyethylene oxide, (j) platelet aggregation inhibitors including cilostazole, aspirin and thienopyridine (ticlopidine, clopidogrel) and GP IIb/IIIa inhibitors such as abciximab, epitifibatide and tirofiban, (k) coagulation pathway modulators including heparinoids such as heparin, low molecular weight heparin, dextran sulfate and β-cyclodextrin tetradecasulfate, thrombin inhibitors such as hirudin, hirulog, PPACK(D-phe-L-propyl-L-arg-chloromethylketone) and argatroban, FXa inhibitors such as antistatin and TAP (tick anticoagulant peptide), Vitamin K inhibitors such as warfarin, as well as activated protein C, (1) cyclooxygenase pathway inhibitors such as aspirin, ibuprofen, flurbiprofen, indomethacin and sulfinpyrazone, (m) natural and synthetic corticosteroids such as dexamethasone, prednisolone, methprednisolone and hydrocortisone, (n) lipoxygenase pathway inhibitors such as nordihydroguairetic acid and caffeic acid, (o) leukotriene receptor antagonists, (p) antagonists of E- and P-selectins, (q) inhibitors of VCAM-1 and ICAM-1 interactions, (r) prostaglandins and analogs thereof including prostaglandins such as PGE1 and PGI2 and prostacyclin analogs such as ciprostene, epoprostenol, carbacyclin, iloprost and beraprost, (s) macrophage activation preventers including bisphosphonates, (t) HMG-CoA reductase inhibitors such as lovastatin, pravastatin, fluvastatin, simvastatin and cerivastatin, (u) fish oils and omega-3-fatty acids, (v) free-radical scavengers/antioxidants such as probucol, vitamins C and E, ebselen, trans-retinoic acid and SOD mimics, (w) agents affecting various growth factors including FGF pathway agents such as bFGF antibodies and chimeric fusion proteins, PDGF receptor antagonists such as trapidil, IGF pathway agents including somatostatin analogs such as angiopeptin and ocreotide, TGF-β pathway agents such as polyanionic agents (heparin, fucoidin), decorin, and TGF-β antibodies, EGF pathway agents such as EGF antibodies, receptor antagonists and chimeric fusion proteins, TNF-α pathway agents such as thalidomide and analogs thereof, Thromboxane A2 (TXA2) pathway modulators such as sulotroban, vapiprost, dazoxiben and ridogrel, as well as protein tyrosine kinase inhibitors such as tyrphostin, genistein and quinoxaline derivatives, (x) MMP pathway inhibitors such as marimastat, ilomastat and metastat, (y) cell motility inhibitors such as cytochalasin B, (z) antiproliferative/antineoplastic agents including antimetabolites such as purine analogs (e.g., 6-mercaptopurine or cladribine, which is a chlorinated purine nucleoside analog), pyrimidine analogs (e.g., cytarabine and 5-fluorouracil) and methotrexate, nitrogen mustards, alkyl sulfonates, ethylenimines, antibiotics (e.g., daunorubicin, doxorubicin), nitrosoureas, cisplatin, agents affecting microtubule dynamics (e.g., vinblastine, vincristine, colchicine, Epo D, paclitaxel and epothilone), caspase activators, proteasome inhibitors, angiogenesis inhibitors (e.g., endostatin, angiostatin and squalamine), rapamycin, cerivastatin, flavopiridol and suramin, (aa) matrix deposition/organization pathway inhibitors such as halofuginone or other quinazolinone derivatives and tranilast, (bb) endothelialization facilitators such as VEGF and RGD peptide, and (cc) blood rheology modulators such as pentoxifylline.

A wide range of therapeutic agent loadings can be used in conjunction with the medical devices of the present invention, with the therapeutically effective amount being readily determined by those of ordinary skill in the art. Typical loadings range, for example, from 1 wt% or less to 2 wt% to 5 wt% to 10 wt% to 25 wt% or more of the polymeric carrier region.

Medical devices having sustained release profiles are beneficial in certain embodiments of the invention. By "sustained release profile" is meant a release profile in which effective amounts of therapeutic agents are released from the medical device to the host tissue or physiological environment over an extended period, such as days, weeks or even months.

As used herein, a "solublizing agent" is a substance which, when added in a sufficient amount, increases the solubility of a therapeutic agent in aqueous solution (e.g., distilled water, physiological saline, phosphate buffered saline, biological fluids, including body fluids such as urine, blood, etc.). Solubility (e.g., as measured on a weight per volume basis) may be increased, for example, by 10% to 25% to 50% to 100% to 250% (2.5 times) to 500% (5 times) to 1000% (10 times) to 2500% (25 times) to 5000% (50 times) to 10000% (100 times) or more. Solubilizing agents meeting these criteria, of course, will also tend to increase the solubility of the therapeutic agent in other water-containing (aqueous) liquids besides those specifically set forth above. Depending on various factors, including the nature of the solublizing agent and the therapeutic agent, the amount of solubilizing agent will vary widely, for example, ranging from 1 wt% or less to 2 wt% to 5 wt% to 10 wt% or more of the polymeric carrier region. The effective amount of employed will be readily determined by those of ordinary skill in the art.

Examples of solubilizing agents include compounds that increase the solubility of the therapeutic agent by intimately associating with the therapeutic agent, for instance, based on one or more of the following non-covalent interactions: electrostatic interactions (e.g, ion-ion, ion-dipole, dipole-dipole), hydrogen bonding interactions, π-π stacking interactions, cation-π interactions, Van der Waals interactions, and hydrophobic effects. These agents typically spontaneously associate with the therapeutic agent in water (e.g., by forming host-guest or other complexes, by self-assembling into micelles, by self-emulsifying, or by spontaneously forming other associations with the therapeutic agent). Many of these agents are amphiphilic (i.e., they have hydrophobic and hydrophilic portions). By increasing the water solubility of the therapeutic agent, the materials tend to increase the transport of the therapeutic agent from the medical device and therefore increase the release of the therapeutic agent.

Examples of solubilizing agents further include compounds that affect the environment in the vicinity of the therapeutic agent (e.g., affect the pH of the environment of the polymeric release region) such that the solubility of the therapeutic agent is increased in that environment.

Specific examples of solubilizing agents include suitable members of the following: (a) host molecules and their derivatives which have internal cavities (including notches, etc.) of molecular dimensions and which may act as hosts for hydrophobic guest molecules, particularly those host molecules having relatively hydrophobic cavities and relatively hydrophilic exteriors, such as macro(poly)cyclic compounds including calixarenes, cylodextrins (e.g., alpha-, beta-, gamma-, delta-, etc. cyclodextrins, and their derivatives, including cationic derivatives and derivatives with hydroxypropyl and sulfobutyl ether groups, among others), crown ethers (and their derivatives), and so forth; (b) amphiphilic dendrimers, including those that form so-called "unimolecular micelles" in water, which typically have a relatively hydrophobic interior and a relatively hydrophilic chain ends, for example, those having interiors selected from poly(aryl ether), poly(amidoamine) (PAMAM), poly(1,4-diaminobutane) (DAB), and poly(propylene imine) and those terminated with hydrophilic end groups or hydrophilic end chains selected from ionic end groups such as carboxylate end groups, poly(ionic monomer) end chains such as polyacrylic acid end chains, poly(hydrophilic monomer) end chains, for instance, polyethylene oxide or polypropylene oxide chains, such as those having from 2 to 4 to 8 to 12 to 20 monomers or more, and so forth; (c) various ionic (e.g., cationic, anionic, zwitterionic) and non-ionic surfactants; mono-, di-, and triglycerides (e.g., glycerol monostearate, glycerol distearate, glycerol monolaurate, etc.); polyhydric alcohol esters; phospholipids, such as lecithin; partial (e.g., mono-, di-, tri-, etc.) fatty acid esters of sugars and sugar alcohols such as sucrose (e.g. sucrose monolaurate and sucrose monostearate, among other sucrose fatty acid esters) and sorbitol (e.g., sorbitan monolaurate, sorbitan monostearate, sorbitan monooleate, sorbitan monopalmitate, sorbitan sesquioleate, sorbitan trioleate, sorbitan tristearate, sorbitan monododecanoate, sorbitan monohexadecanoate, sorbitan monooctadecanoate, sorbitan trioctadecanoate, sorbitan mono-9-monodecenoate, and sorbitan tri-9-octadecenoate, among other sorbitan fatty acid esters); fatty alcohol ethers of oligoglucosides (e.g., akylpolyglucosides); polyoxyalkylenes such as polyoxyethylene and polyoxypropylene as well as their derivatives and copolymers (note that polyoxyethylene, polyoxyethylene ether, polyethylene glycol and polyethylene oxide are often used synonymously in the art), for example, polyoxyethylene-polyoxypropylene block copolymers (also known as poloxamers) and polyoxyalkylene derivatives such as polyoxyalkylene esters, including polyethylene glycol esters, polypropylene glycol esters, polyoxyalkylene sorbitan esters such as polyethoxylated fatty acid esters of sorbitan (e.g., polysorbates), and fatty acid esters of polyethylene oxide (e.g., polyoxyethylene stearates), polyoxyalkylene ethers, including fatty alcohol ethers of polyethylene oxide (e.g., polyoxyethylated lauryl ether) and alkylphenol ethers of polyethylene oxide (e.g., polyethoxylated octylphenol), and ethoxylated fats and oils (e.g., ethoxylated castor oil and polyoxyethylated castor oil, also known as polyethylene glycol-glyceryl triricinoleate, and so forth; (d) pH control agents, particularly where the low-solubility therapeutic agents have acidic and/or basic groups which may be ionized using such agents to increase water solubility, such as buffers as well as organic and inorganic acids, bases and their salts, for example, ascorbic, benzoic, edetic, sebacic, sorbic, glutamic, p-toluenesulfonic, citric, succinic, fumaric, adipic, malic and tartaric acids, meglumine, monoethanolamine, diethanolamine, triethanolamine, triazine base, guanidine, N-methyl glucamine, sodium citrate, potassium citrate, alkali and alkaline earth metal salts of carbonate and bicarbonate, such as sodium bicarbonate, and sodium phosphate (di-basic and mono-basic), among many others; and (e) mixtures of the foregoing. For further examples of solubilizing agents, see, .e.g., U.S. Patent Appln. Nos. 2005/0186276 to Berchielli et al., 2004/0053894 to Mazess et al., and 2002/0006443 to Curatolo et al. see also F. Aulenta et al., "Dendrimers: a new class of nanoscopic containers and delivery devices," European Polymer Journal 39 (2003) 1741-1771; and M. Liu and J.M.M. Frechet; "Designing dendrimers for drug delivery," PSTT Vol. 2, No. 10 (October 1999) 393-401.

As noted above, a "polymeric" region is one that contains polymers, for example, 50 wt% or lower to 75 wt% to 90 wt% to 95 wt% to 97.5 wt% to 99 wt% polymers, or more.

As used herein, "polymers" are molecules containing multiple copies (e.g., from 2 to 5 to 10 to 25 to 50 to 100 to 250 to 500 to 1000 or more copies) of one or more constitutional units, commonly referred to as monomers.

Polymers may take on a number of configurations, which may be selected, for example, from cyclic, linear and branched configurations. Branched configurations include star-shaped configurations (e.g., configurations in which three or more chains emanate from a single branch point), comb configurations (e.g., configurations having a main chain and a plurality of side chains), dendritic configurations (e.g., arborescent and hyperbranched polymers), and so forth.

As used herein, "homopolymers" are polymers that contain multiple copies of a single constitutional unit. "Copolymers" are polymers that contain multiple copies of at least two dissimilar constitutional units, examples of which include random, statistical, gradient, periodic (e.g., alternating) and block copolymers.

As used herein, "block copolymers" are copolymers that contain two or more polymer blocks that differ in composition, for instance, because a constitutional unit (i.e., monomer) is found in one polymer block that is not found in another polymer block. As used herein, a "polymer block" is a grouping of constitutional units (e.g., 5 to 10 to 25 to 50 to 100 to 250 to 500 to 1000 or more units). Blocks can be branched or unbranched. Blocks can contain a single type of constitutional unit (also referred to herein as "homopolymeric blocks") or multiple types of constitutional units (also referred to herein as "copolymeric blocks") which may be provided, for example, in a random, statistical, gradient, or periodic (e.g., alternating) distribution.

Polymers for use in the present invention may be selected, for example, from suitable members of the following: polycarboxylic acid polymers and copolymers including polyacrylic acids; acetal polymers and copolymers; acrylate and methacrylate polymers and copolymers (e.g., n-butyl methacrylate); cellulosic polymers and copolymers, including cellulose acetates, cellulose nitrates, cellulose propionates, cellulose acetate butyrates, cellophanes, rayons, rayon triacetates, and cellulose ethers such as carboxymethyl celluloses and hydroxyalkyl celluloses; polyoxymethylene polymers and copolymers; polyimide polymers and copolymers such as polyether block imides and polyether block amides, polyamidimides, polyesterimides, and polyetherimides; polysulfone polymers and copolymers including polyarylsulfones and polyethersulfones; polyamide polymers and copolymers including nylon 6,6, nylon 12, polycaprolactams and polyacrylamides; resins including alkyd resins, phenolic resins, urea resins, melamine resins, epoxy resins, allyl resins and epoxide resins; polycarbonates; polyacrylonitriles; polyvinylpyrrolidones (cross-linked and otherwise); polymers and copolymers of vinyl monomers including polyvinyl alcohols, polyvinyl halides such as polyvinyl chlorides, ethylene-vinyl acetate copolymers (EVA), polyvinylidene chlorides, polyvinyl ethers such as polyvinyl methyl ethers, polystyrenes, styrene-maleic anhydride copolymers, vinyl-aromatic-alkylene copolymers, including styrene-butadiene copolymers, styrene-ethylene-butylene copolymers (e.g., a polystyrene-polyethylene/butylene-polystyrene (SEBS) copolymer, available as Kraton® G series polymers), styrene-isoprene copolymers (e.g., polystyrene-polyisoprene-polystyrene), acrylonitrile-styrene copolymers, acrylonitrile-butadiene-styrene copolymers, styrene-butadiene copolymers and styrene-isobutylene copolymers (e.g., polyisobutylene-polystyrene and polystyrene-polyisobutylene-polystyrene block copolymers such as those disclosed in U.S. Patent No. 6,545,097 to Pinchuk), polyvinyl ketones, polyvinylcarbazoles, and polyvinyl esters such as polyvinyl acetates; polybenzimidazoles; ethylene-methacrylic acid copolymers and ethylene-acrylic acid copolymers, where some of the acid groups can be neutralized with either zinc or sodium ions (commonly known as ionomers); polyalkyl oxide polymers and copolymers including polyethylene oxides (PEO); polyesters including polyethylene terephthalates and aliphatic polyesters such as polymers and copolymers of lactide (which includes lactic acid as well as d-,1- and meso lactide), epsilon-caprolactone, glycolide (including glycolic acid), hydroxybutyrate, hydroxyvalerate, para-dioxanone, trimethylene carbonate (and its alkyl derivatives), 1,4-dioxepan-2-one, 1,5-dioxepan-2-one, and 6,6-dimethyl-1,4-dioxan-2-one (a copolymer of poly(lactic acid) and poly(caprolactone) is one specific example); polyether polymers and copolymers including polyarylethers such as polyphenylene ethers, polyether ketones, polyether ether ketones; polyphenylene sulfides; polyisocyanates; polyolefin polymers and copolymers, including polyalkylenes such as polypropylenes, polyethylenes (low and high density, low and high molecular weight), polybutylenes (such as polybut-1-ene and polyisobutylene), polyolefin elastomers (e.g., santoprene), ethylene propylene diene monomer (EPDM) rubbers, poly-4-methyl-pen-1-enes, ethylene-alpha-olefin copolymers, ethylene-methyl methacrylate copolymers and ethylene-vinyl acetate copolymers; fluorinated polymers and copolymers, including polytetrafluoroethylenes (PTFE), poly(tetrafluoroethylene-co-hexafluoropropene) (FEP), modified ethylene-tetrafluoroethylene copolymers (ETFE), and polyvinylidene fluorides (PVDF); silicone polymers and copolymers; thermoplastic polyurethanes (TPU); elastomers such as elastomeric polyurethanes and polyurethane copolymers (including block and random copolymers that are polyether based, polyester based, polycarbonate based, aliphatic based, aromatic based and mixtures thereof; examples of commercially available polyurethane copolymers include Bionate®, Carbothane®, Tecoflex®, Tecothane®, Tecophilic®, Tecoplast®, Pellethane®, Chronothane® and Chronoflex®); p-xylylene polymers; polyiminocarbonates; copoly(ether-esters) such as polyethylene oxide-polylactic acid copolymers; polyphosphazines; polyalkylene oxalates; polyoxaamides and polyoxaesters (including those containing amines and/or amido groups); polyorthoesters; biopolymers, such as polypeptides, proteins, polysaccharides and fatty acids (and esters thereof), including fibrin, fibrinogen, collagen, elastin, chitosan, gelatin, starch, glycosaminoglycans such as hyaluronic acid; as well as blends and further copolymers of the above.

Further examples of polymers for use in the present invention, which are not necessarily exclusive of those above, may be selected, for example, from suitable members of the following: ethylenic copolymers including ethylene vinyl acetate copolymers (EVA) and copolymers of ethylene with acrylic acid or methacrylic acid; elastomeric polyurethanes and polyurethane copolymers; metallocene catalyzed polyethylene (mPE) and mPE copolymers; vinyl aromatic copolymers; polyester-ether copolymers, polyamide-ether copolymers; silicone; and mixtures of the same.

Among vinyl aromatic copolymers are included polyvalence-poly(vinyl aromatic) block copolymers such as those containing (a) one or more polyalkylene homopolymer or copolymer blocks, which may contain one or more of ethylene, butylene and isobutylene, and (b) one or more poly(vinyl aromatic)homopolymer or copolymer blocks, which may contain one or more of styrene and alpha-methyl-styrene, for example, block copolymers of polyisobutylene with polystyrene or polymethylstyrene, such as polystyrene-polyisobutylene-polystyrene triblock copolymers, among others. These polymers are described, for example, in U.S. Patent No. 6,545,097 to Pinchuk et al.

Elastomeric polyurethanes are a family of polymers that are synthesized from polyfunctional isocyanates (e.g., diisocyanates, including both aliphatic and aromatic diisocyanates) and polyols (also, referred to as macroglycols, e.g., macrodiols). Commonly employed macroglycols include polyester glycols, polyether glycols and polycarbonate glycols. Typically, aliphatic or aromatic diols are also employed as chain extenders. Polyurethanes are commonly classified based on the type of macroglycol employed, with those containing polyester glycols being referred to as polyester polyurethanes, those containing polyether glycols being referred to as polyether polyurethanes, and those containing polycarbonate glycols being referred to as polycarbonate polyurethanes. Polyurethanes are also commonly designated as aromatic or aliphatic on the basis of the chemical nature of the diisocyanate component in their formulation. Examples of commercially available polyurethane copolymers include Carbothane® (an aliphatic polycarbonate polyurethane), Tecoflex® (an aliphatic polyether polyurethane), Tecothane® (an aromatic polyether polyurethane), Tecophilic® (an aliphatic polyether polyurethane), Tecoplast®(an aromatic polyether polyurethane), Pellethane® (an aromatic polyether polyurethane), Chronothane® (an aromatic polyether polyurethane) and Chronoflex® (an aliphatic polycarbonate polyurethane), among many others.

Among polyether-polyamide block copolymers are included those containing (a) one or more polyethers blocks selected from homopolymer and copolymer blocks containing one or more of ethylene oxide, trimethylene oxide, propylene oxide and tetramethylene oxide, (b) one or more polyamide blocks selected from nylon homopolymer and copolymer blocks such as nylon 6, nylon 4/6, nylon 6/6, nylon 6/10, nylon 6/12, nylon 11 and nylon 12 blocks. A specific example is poly(tetramethylene oxide)-nylon-12 block copolymer, available from Elf Atochem as PEBAX.

Among ethylene vinyl acetate copolymers are included random copolymers having a vinyl acetate weight percent ratio of from about 0.5% to 1% to 2% to 5% to about 15% to about 20% to about 30% to about 40%. In general, the higher the vinyl acetate content, the lower the stiffness and Durometer of the EVA. Hence, the stiffness and durometer may be varied within the device, in certain embodiments. Taking a ureteral stent as an example, a stent may be produced having distinct end regions of different durometer value with a transitional region in between.

The release profile of the therapeutic agent from the polymeric region will be affected by a number of variables including the specific therapeutic agent(s), solublizing agent(s) and polymer(s) that are selected and their relative amounts. The release profile will also be affected by the size, number and/ or position of the polymeric carrier regions within the device. For example, the release profile may be modified by varying the thickness or surface area of the polymeric region. Moreover, multiple polymeric carrier regions may be employed. For example, multiple polymeric carrier regions having the same or different content (e.g., different polymer, solubilizing agent and/or therapeutic agent content) may be positioned laterally with respect to one another. Alternatively, a polymeric layer (e.g., formed from one or more polymers described above, either with or without solubilizing and therapeutic agents) may be positioned over a polymeric carrier region in accordance with the invention, thereby acting as a barrier layer.

In some embodiments, the release profile may be modified by increasing the rate at which the polymeric region absorbs water from the surrounding environment, for example, by adding a rapidly hydrating polymer or polymer block (or by increasing the ratio of a rapidly hydrating polymer or polymer block vis-à-vis a slowly hydrating polymer or polymer block), by the addition of an osmotic agent such as a soluble salt excipient, and so forth.

Numerous techniques are available for forming polymeric carrier regions in accordance with the present invention.

For example, where the polymeric carrier region is formed from one or more polymers having thermoplastic characteristics, a variety of standard thermoplastic processing techniques may be used to form the polymeric carrier region, including compression molding, injection molding, blow molding, spinning, vacuum forming and calendaring, extrusion into sheets, fibers, rods, tubes and other cross-sectional profiles of various lengths, and combinations of these processes. Using these and other thermoplastic processing techniques, entire devices or portions thereof can be made.

Mixing or compounding the one or more polymer(s), one or more therapeutic agent(s), and one or more solubilizing agent(s) during processing may be performed using any technique known in the art. For example, where thermoplastic materials are employed, a polymer melt may be formed which includes (a) the polymer(s) and (b) the therapeutic agent(s), the solubilizing agent(s), or both. A common way of doing so is to apply mechanical shear to a mixture of the polymer(s) and the therapeutic agent(s) and/or solubilizing agent(s). Devices in which these materials may be mixed in this fashion include devices such as single screw extruders, twin screw extruders, banbury mixers, high-speed mixers, and ross kettles, among others.

Any of the polymer(s), therapeutic agent(s), and solubilizing agent(s) may be precompounded or individually premixed to facilitate subsequent processing. For example, a therapeutic agent may be precompounded with a polymer and then compounded with a solubilizing agent, or a solubilizing agent may be precompounded with a polymer and subsequently compounded with a therapeutic agent. As another alternative, a therapeutic agent may be preblended with a solubilizing agent (e.g., by micronizing and blending, by dissolving and drying, e.g., by spray drying, and so forth) before being compounded with a polymer, thereby intimately association of the therapeutic agent and solubilizing agent. Of course, the polymer(s), therapeutic agent(s), and solubilizing agent(s) may all be mixed and compounded in a single step as noted above.

Once compounded, the materials can then be processed using any of a variety of thermoplastic processing techniques such as those described above (e.g., extrusion, molding, casting, etc.). Among the processing conditions that may be controlled during processing are the temperature, applied shear rate and residence time in the processing device.

Other processing techniques besides thermoplastic processing techniques may also be used to form the polymeric carrier regions of the present invention, including solvent-based techniques. Using these techniques, a polymeric carrier region can be formed by (a) first providing a solution or dispersion that contains the polymer(s), therapeutic agent(s), and solubilizing agent(s), and (b) subsequently removing the solvent. The solvent that is ultimately selected will contain one or more solvent species, which are generally selected based on their ability to dissolve the polymer(s) that form the polymeric carrier region (and in many embodiments the therapeutic agent(s) and solubilizing agent(s) as well), in addition to other factors, including drying rate, surface tension, etc. Preferred solvent-based techniques include, but are not limited to, solvent casting techniques, spin coating techniques, web coating techniques, solvent spraying techniques, dipping techniques, techniques involving coating via mechanical suspension including air suspension, ink jet techniques, electrostatic techniques, and combinations of these processes.

In some embodiments of the invention, a polymer containing solution (where solvent-based processing is employed) or a polymer melt (where thermoplastic processing is employed) is applied to a substrate to form a polymeric carrier region. For example, the substrate can correspond to all or a portion of an implantable or insertable medical device to which a polymeric carrier region is applied. The substrate can also be, for example, a template, such as a mold, from which the polymeric carrier region is removed after solidification. In other embodiments, for example, extrusion and co-extrusion techniques, one or more polymeric carrier regions are formed without the aid of a substrate. In a more specific example, an entire stent body is extruded. In another, a polymeric layer is co-extruded along with and underlying stent body. In another, a polymeric layer is provided on an underlying step body by spraying or extruding a coating layer onto a pre-existing stent body. In yet another more specific example, a stent is cast in a mold.

As noted above, polymeric barrier layers may be provided over the polymeric carrier region in accordance with an embodiment of the invention. In these embodiments, the polymeric barrier layer may be formed over the polymeric carrier region, for example, using one of the solvent based or thermoplastic techniques described above. Alternatively, a previously formed polymeric barrier region may be adhered over a polymeric carrier region.

In other embodiments, medical devices in accordance with the invention may contain further layers such as a layer containing a radio-opacifying agent and/or a layer on an external surface that provides lubricity. Such a lubricious layer may be desirable, for example, to facilitate insertion and implantation of the medical device and include various lubricious hydrogels known in the art.

### EXAMPLE

Ureteral stents are used, for example, in post endourological procedures to act as a scaffold in the event of ureteral obstruction secondary to the procedure. Stents are also used as palliative devices to provide patency in the presence of congenital defects, strictures or malignancies that cause a ureter obstruction. The ureteral stents of the present Example are formed based on the design of the Percuflex® Ureteral Stent, which is commercially available from Boston Scientific, Natick, MA, USA.

A schematic diagram of such a stent 10 is illustrated in Fig. 1. The stent 10 is a tubular polymer extrusion containing a renal pigtail 12, a shaft 14 and a bladder pigtail 16. The stent 10 is inserted into the ureter to provide ureteral rigidity and allow the passage of urine. The pigtails 12, 16 serve to keep the stent 10 in place once positioned by the physician. The stent 10 is further provided with the following: (a) a tapered tip 11, to aid insertion, (b) multiple side ports 18 (one numbered), which are arranged in a spiral pattern down the length of the body to promote drainage, (c) graduation marks 20 (one illustrated), which are used for visualization by the physician to know when the appropriate length of stent has been inserted into the ureter, and (d) a Nylon suture 22, which aids in positioning and withdrawal of the stent, as is known in that art. During placement, such ureteral stents 10 are typically placed over a urology guidewire, through a cystoscope and advanced into position with a positioner. Once the proximal end of the stent is advanced into the kidney/renal calyx, the guidewire is removed, allowing the pigtails 12, 16 to form in the kidney and bladder.

Unlike the above Percuflex® Ureteral Stent, however, the stents used in the present Example contain a low-solubility therapeutic agent such as triclosan and a solubilizing agent such as one or more of the following: a) surfactants such as cationic surfactants (e.g., those having fatty amine salts or ammonium salts such as alkyl pyridinium and quaternary ammonium, among others), anionic surfactants (e.g., sodium lauryl sulfate, among others), and nonionic surfactants (e.g., Tween-80, among others), b) pluronics (such as pluronic F127 and F108, among others), c) amino acids such as L-arginine, and the amino sugar alcohol N-methylglucamine, among others, d) amphiphilic molecules or gels (e.g., cyclodextrin, among others), e) amphoteric surfactants (e.g., betaines, among others), f) ampholytic surfactants (e.g., N-alkyl propionic acids, among others), g) surfactant polymers (e.g., hydrophobically modified polymers such as PEG and PEO, among others), and h) zwitterionics (e.g., sulfobetaines types and dimethylalkylammoniopropanesulfonates, among others).

A nonionic, broad spectrum, antimicrobial agent, triclosan has been used for more than twenty years in a variety of personal care products such as shower gels, soaps, mouthwash and toothpaste, detergents, lotions, creams, and cosmetics. It is also incorporated into plastic toys, polymers, and textiles. Recently, triclosan has been incorporated as an antimicrobial agent in a biodegradable coated VICRYL Plus antimicrobial Suture from Ethicon, Inc., a Johnson & Johnson Company. The antimicrobial effectiveness of triclosan is well documented and described to be immediate, persistent and broad-spectrum against most gram positive and gram negative aerobic and anaerobic bacteria, some yeast and fungi, even at a very low concentration (MIC < 0.3 ppm) in most organisms tested. See, Bhargava, H.N. et al., "Triclosan: Application and safety." AJIC American Journal of Infection Control, vol. 24(3) June 1996, pp. 209-218; Jones, RD et al., "Triclosan: a review of effectiveness and safety in health care settings." Am J lnfect Control 2000 Apr; 28(2):184-96; Regos, J., "Antimicrobial spectrum of triclosan, a broad-spectrum antimicrobial agent for topical application. II. Comparison with some other antimicrobial agent." Dermatologia 1979; 158(1): 72-9.

The triclosan-containing ureteral stents in accordance with the present invention may contain the following ingredients in the following amounts: (a) 0.1 to 30 wt% triclosan (available under the trade name Irgacare™ MP from Ciba Specialty Chemicals), (b) 5 to 30 wt% bismuth subcarbonate (Mallinckrodt) as a radio-opacifying agent, (b) an effective amount of the solubilizing agent, and (c) the balance ethylene vinyl acetate copolymer (Elvax 460, from DuPont). First, the triclosan and solubilizing agent are preblended, for example, premixed in powder form, micronized and mixed, or dissolved and dried (e.g., freeze-dried, air dried, oven dried, vacuum dried, spray dried, etc.). The preblended triclosan and solubilizing agent are then compounded with the EVA copolymer, for example, in twin screw extruder with a low-shear profile screw design. The barrel temperature, screw speed and throughput are adjusted so as to achieve uniform mixing and avoid clumping and possible degradation. After compounding, pellets are extruded into tubes of an appropriate diameter, for example, in a standard 1" screw diameter extruder with a 24:1 L/D, 3:1 compression ratio, low shear screw. The barrel temperature, screw speed and throughput are adjusted so as to achieve uniform mixing and avoid clumping and possible degradation.

The extruded material is then cut, provided with a tapered tip, annealed, marked with ink (Formulab CFX-00), and coated with a coating of Hydroplus™ (a hydrophilic polyacrylic acid polymer coating available from Boston Scientific Corp., Natick, Mass. and described in U.S. Pat. No. 5,091,205), followed by side port formation, pigtail formation by hot air treatment, and the addition of a suture, as is known in the art.

## Claims

1. A medical device comprising a polymeric carrier region that comprises a polymer, a therapeutic agent, and a solubilising agent selected from host molecules which have internal cavities of molecular dimensions, amphiphilic dedrimers and ionic or non-ionic surfactants, said medical device being adapted for implantation or insertion into a subject's body and said therapeutic agent having a solubility at 37°C of less than 1 mg/ml in body fluid present at the site of implantation, wherein said solubilising agent increases the solubility of said therapeutic agent, wherein said medical device is a urological medical device and said body fluid is urine, and wherein said polymeric carrier region comprises an ethylene vinyl acetate copolymer having a vinyl acetate content ranging from 0.5 to 40%.

2. The medical device of claim 1, wherein said urological medical device is selected from a ureteral stent, a urinary catheter, a sling, an artificial bladder, an artificial sphincter, an erectile dysfunction implant, and an intrauterine device.

3. The medical device of claim 1, comprising a plurality of said polymeric carrier regions.

4. The medical device of claim 1, wherein said polymeric carrier region corresponds to an entire medical device or to an entire component of a medical device.

5. The medical device of claim 1, wherein said polymeric carrier region is in the form of a layer that at least partially covers an underlying substrate.

6. The medical device of clam 1, wherein said polymeric carrier region comprises a therapeutic agent selected from antiproliferative agents, vascular cell growth promoters, antimicrobial agents, analgesic agents, immune-suppression agents, anti-inflammatory agents, antispasmodic agents, alpha blockers, calcium channel blockers, beta agonists, neoplastic agents, and cytostatic agents.

7. The medical device of claim 1, wherein said polymeric carrier region comprises a therapeutic agent selected from paclitaxel and triclosan.

8. The medical device of claim 1, wherein said polymeric carrier region comprises a solubilising agent that increases the solubility of said therapeutic agent by at least ten times, or that alters the pH environment around said therapeutic agent.

9. The medical device of claim 1, wherein said polymeric carrier region comprises a solubilising agent selected from an acid, an acid salt, a base, a base salt and combinations thereof.

10. The medical device of claim 1, wherein said polymeric carrier region comprises a solubilising agent that non-covalently binds to or encapsulates said therapeutic agent.

11. The medical device of claim 1, wherein said polymeric carrier region comprises a solubilising agent that spontaneously forms a water soluble complex, micelle, or emulsion upon exposure to said body fluid.

12. The medical device of claim 1, wherein said polymeric carrier region comprises a solubilising agent that forms a host-guest complex with said therapeutic agent.

13. The medical device of claim 1, wherein said polymeric carrier region comprises a solubilising agent that is selected from fatty acid mono-, di-, and tri-glycerides, polyhydric alcohol esters, phospholipids, fatty acid esters of sugars and sugar alcohols, fatty alcohol ethers of oligoglucosides, polyoxyalkylene, polyoxyalkylene copolymers, polyoxyalkylene esters, polyoxyalkylene ethers, and combinations thereof.

## Patentansprüche

1. Medizinische Vorrichtung, aufweisend einen polymeren Trägerbereich, der ein Polymer, ein therapeutisches Mittel und ein Lösungshilfsmittel, das ausgewählt ist aus Wirtsmolekülen, die innere Hohlräume mit molekularen Abmessungen haben, amphiphilen Dendrimeren und ionischen oder nichtionischen Tensiden, aufweist, welche medizinische Vorrichtung ausgestaltet ist zur Implantierung oder zum Einführen in den Körper eines Subjekts, wobei das therapeutische Mittel eine Löslichkeit bei 37°C von weniger als 1 mg/ml in Körperfluid, das an dem Ort der Implantierung vorhanden ist, hat, wobei das Lösungshilfsmittel die Löslichkeit des therapeutischen Mittels erhöht, wobei die medizinische Vorrichtung eine urologische medizinische Vorrichtung ist und das Körperfluid Urin ist, und wobei der polymere Trägerbereich ein Ethylen-Vinylacetat-Copolymer mit einem Vinylacetatgehalt im Bereich von 0,5 bis 40% aufweist.

2. Medizinische Vorrichtung nach Anspruch 1, bei der die urologische medizinische Vorrichtung ausgewählt ist aus einem Harnleiterstent, einem Harnkatheter, einer Schlinge, einer künstlichen Blase, einem künstlichen Schließmuskel, einem Schwelldisfunktionsimplantat und einer Intrauterinvorrichtung.

3. Medizinische Vorrichtung nach Anspruch 1, aufweisend mehrere polymere Trägerbereiche.

4. Medizinische Vorrichtung nach Anspruch 1, bei der der polymere Trägerbereich einer gesamten medizinischen Vorrichtung oder einer gesamten Komponente einer medizinischen Vorrichtung entspricht.

5. Medizinische Vorrichtung nach Anspruch 1, bei der der polymere Trägerbereich die Form einer Schicht hat, die zumindest teilweise ein darunterliegendes Substrat bedeckt.

6. Medizinische Vorrichtung nach Anspruch 1, bei der der polymere Trägerbereich ein therapeutisches Mittel aufweist, das ausgewählt ist aus Antiproliferationsmitteln, Wuchsbeschleunigern für vaskuläre Zellen, antimikrobiellen Mitteln, analgetischen Mitteln, Immununterdrückungsmitteln, entzündungshemmenden Mitteln, krampflösenden Mitteln, Alphablockern, Kalziumkanalblockern, Beta-Agonisten, neoplastischen Mitteln und cytostatischen Mitteln.

7. Medizinische Vorrichtung nach Anspruch 1, bei der der polymere Trägerbereich ein therapeutisches Mittel aufweist, das ausgewählt ist aus Paclitaxel und Triclosan.

8. Medizinische Vorrichtung nach Anspruch 1, bei der der polymere Trägerbereich ein Lösungshilfsmittel aufweist, das die Löslichkeit des therapeutischen Mittels um zumindest das Zehnfache erhöht oder das das pH-Umfeld um das therapeutische Mittel herum ändert.

9. Medizinische Vorrichtung nach Anspruch 1, bei der der polymere Trägerbereich ein Lösungshilfsmittel aufweist, das aus einer Säure, einem sauren Salz, einer Base, einem basischen Salz und Kombinationen hiervon ausgewählt ist.

10. Medizinische Vorrichtung nach Anspruch 1, bei der der polymere Trägerbereich ein Lösungsmittel aufweist, das das therapeutische Mittel nichtkovalent bindet oder einkapselt.

11. Medizinische Vorrichtung nach Anspruch 1, bei der der polymere Trägerbereich ein Lösungshilfsmittel aufweist, das spontan einen/eine wasserlöslichen Komplex, Mizelle oder Emulsion bildet, wenn es dem Körperfluid ausgesetzt wird.

12. Medizinische Vorrichtung nach Anspruch 1, bei der der polymere Trägerbereich ein Lösungshilfsmittel aufweist, das einen Wirt-Gast-Komplex mit dem therapeutischen Mittel bildet.

13. Medizinische Vorrichtung nach Anspruch 1, bei der der polymere Trägerbereich ein Lösungshilfsmittel aufweist, das ausgewählt ist aus Fettsäure-Mono-, -Di- und -Triglyzeriden, polyhydrischen Alkoholestern, Phospholipiden, Fettsäureestern von Zuckern und Zuckeralkoholen, Fettalkoholethern von Oligoglucosiden, Polyoxyalkylenen, Polyoxyalkylen-Copolymeren, Polyoxyalkylen-Estern, Polyoxyalkylen-Ethern und Kombinationen von diesen.

## Revendications

1. Dispositif médical comprenant une région de support polymère qui comprend un polymère, un agent thérapeutique et un agent de solubilisation choisi parmi des molécules hôtes qui comportent des cavités internes de dimensions moléculaires, des dendrimères amphiphiles et des agents tensioactifs ioniques ou non ioniques, ledit dispositif médical étant adapté pour une implantation ou une insertion à l'intérieur du corps d'un sujet et ledit agent thérapeutique présentant une solubilité à 37°C inférieure à 1 mg/ml dans un fluide du corps présent au niveau du site d'implantation, dans lequel ledit agent de solubilisation augmente la solubilité dudit agent thérapeutique, dans lequel ledit dispositif médical est un dispositif médical urologique et ledit fluide du corps est l'urine, et dans lequel ladite région de support polymère comprend un copolymère éthylène et acétate de vinyle présentant une teneur en acétate de vinyle s'inscrivant dans la plage de 0,5 à 40%.

2. Dispositif médical selon la revendication 1, dans lequel ledit dispositif médical urologique est choisi parmi une endoprothèse urétérale, un cathéter urinaire, une fronde/bandelette, une vessie artificielle, un sphincter artificiel, un implant pour dysfonctionnement érectile et un dispositif intra-utérin.

3. Dispositif médical selon la revendication 1, comprenant une pluralité desdites régions de support polymère.

4. Dispositif médical selon la revendication 1, dans lequel ladite région de support polymère correspond à un dispositif médical complet ou à un composant complet d'un dispositif médical.

5. Dispositif médical selon la revendication 1, dans lequel ladite région de support polymère se présente sous la forme d'une couche qui recouvre au moins partiellement un substrat sous-jacent.

6. Dispositif médical selon la revendication 1, dans lequel ladite région de support polymère comprend un agent thérapeutique choisi parmi les agents anti-proliférants, les promoteurs de croissance de cellules vasculaires, les agents antimicrobiens, les agents analgésiques, les agents d'immunosuppression, les agents anti-inflammatoires, les agents antispasmodiques, les alpha-bloquants, les inhibiteurs des canaux calciques, les bêta-agonistes, les agents néoplasiques et les agents cytostatiques.

7. Dispositif médical selon la revendication 1, dans lequel ladite région de support polymère comprend un agent thérapeutique choisi parmi le paclitaxel et le triclosan.

8. Dispositif médical selon la revendication 1, dans lequel ladite région de support polymère comprend un agent de solubilisation qui augmente la solubilité dudit agent thérapeutique d'un facteur d'au moins dix ou qui altère l'environnement de pH au voisinage dudit agent thérapeutique.

9. Dispositif médical selon la revendication 1, dans lequel ladite région de support polymère comprend un agent de solubilisation choisi parmi un acide, un sel d'acide, une base, un sel de base et des combinaisons afférentes.

10. Dispositif médical selon la revendication 1, dans lequel ladite région de support polymère comprend un agent de solubilisation qui se lie de façon non covalente audit agent thérapeutique ou qui l'encapsule.

11. Dispositif médical selon la revendication 1, dans lequel ladite région de support polymère comprend un agent de solubilisation qui forme spontanément un complexe soluble à l'eau, une micelle ou une émulsion suite à une exposition audit fluide du corps.

12. Dispositif médical selon la revendication 1, dans lequel ladite région de support polymère comprend un agent de solubilisation qui forme un complexe hôte-invité avec ledit agent thérapeutique.

13. Dispositif médical selon la revendication 1, dans lequel ladite région de support polymère comprend un agent de solubilisation qui est choisi parmi les mono-glycérides, di-glycérides et triglycérides d'acide gras, les esters d'alcool polyhydrique, les phospholipides, les esters d'acide gras de sucres et d'alcools de sucre, les éthers d'alcool gras d'oligoglucosides, le polyoxyalkylène, les copolymères de polyoxyalkylène, les esters de polyoxyalkylène, les éthers de polyoxyalkylène, et des combinaisons afférentes.
